(19) Europäisches Patentamt

European Patent Office

Office européen des brevets

(11) Veröffentlichungsnummer : **0 007 393**
**B1**

(12) **EUROPÄISCHE PATENTSCHRIFT**

(45) Veröffentlichungstag der Patentschrift :
11.02.81

(21) Anmeldenummer : 79101426.9

(22) Anmeldetag : 10.05.79

(51) Int. Cl.³ : **A 61 B 17/18**

(54) **Dem Längen- und Dickenwachstum nachgebendes Implantat.**

(30) Priorität : 16.06.78 CH 6575/78

(43) Veröffentlichungstag der Anmeldung :
06.02.80 (Patentblatt 80/03)

(45) Bekanntmachung des Hinweises auf die Patenterteilung : 11.02.81 Patentblatt 81/06

(84) Benannte Vertragsstaaten :
AT DE FR GB

(56) Entgegenhaltungen :
US - A - 2 501 978

(73) Patentinhaber : GEBRDER SULZER AKTIENGESELL-
SCHAFT
Zürcherstrasse 9
CH-8401 Winterthur (CH)

(72) Erfinder : Scherrer, Hans Gebhard, Dr.-med.
Klinik Wilhelm Schulthess
CH-8008 Zürich (CH)

(74) Vertreter : Sparing, Nikolaus, Dipl.-Ing.
Lindemannstrasse 31
D-4000 Düsseldorf (DE)

« Dem Längen- und Dickenwachstum nachgebendes Implantat »

Die Erfindung betrifft ein dem Längen- und Dickenwachstum nachgebendes Implantat zur Stützung von Röhrenknochen des menschlichen Skeletts.

Bei einer Gruppe von regionalen oder generalisierten Systemerkrankungen des Skeletts, den sogenannten Skelett-Dysplasien, die bei Heranwachsenden auftreten, führt das Wachstum der Röhrenknochen des menschlichen Skeletts auch ohne äußere Belastungen zu Verkrümmungen und Verbiegungen der Knochen, wobei variable Knochendichten und Knochenfestigkeiten und eine erhöhte Brüchigkeit der Knochen auftreten ; diese Fehlentwicklungen führen häufig zu Spontan-Frakturen der betroffenen Knochen.

Zur Behandlung dieser Krankheiten, beispielsweise auch mit Hilfe der Osteotomie, und/oder zur Stützung dieser Knochen und zur Führung ihres Wachstums sind bisher beispielsweise sogenannte Osteotomie-Platten angewendet worden, die von außen auf den Knochen bzw. dessen Teilstücke aufgeschraubt worden sind ; eine andere Art von dafür benutzten Implantaten sind den Knochen in Längsrichtung, zum Zeitpunkt der Implantation möglichst vollständig durchsetzende Marknägel, die als « Armierung » in den Knochen eingezogen werden. Diese beiden Arten der Implantation können dem Wachstum des Knochens in keiner Weise Rechnung tragen. Innerhalb kurzer Zeit müssen sie daher reoperiert und ersetzt werden, da beispielsweise die über den Nagel hinauswachsenden Knochen - vor allem an ihrem distalen Ende - sofort wieder in ein gekrümmtes Wachstum übergehen ; die Platten müssen unter Umständen schon nach einigen Monaten ausgewechselt werden, während die marknägel im allgemeinen etwa 1 1/2 bis 2 1/2 Jahre implantiert bleiben können, ohne ersetzt zu werden.

Als Weiterentwicklung des Marknagels wird daher ein Marknagel aus zwei teleskopartig ineinander greifenden Teilen benutzt, durch den dem Längenwachstum Rechnung getragen werden soll ; wegen zu großer Reibung und Verklemmen der teleskopartig ineinandergreifenden Teile ist dieses Ziel jedoch häufig nicht zu erreichen.

Ein weiterer Nachteil der beiden Marknagel-Implantate besteht darin, daß sie den Knochen starr fixieren und praktisch von jeder Belastung befreien. Dies führt zu Abbauerscheinungen und zu erhöhter Frakturneigung des Knochenmantels, weil dieser nicht mehr beansprucht wird. Darüberhinaus wird durch die Marknägel der Knochenstoffwechsel gestört, so daß es zu einem vermehrten Knochenabbau und zu einem verhinderten Knochenaufbau kommt ; zusätzlich ist durch die Marknägel auch die Gefäßversorgung vom Markraum her beeinträchtigt.

Spezielle Probleme mit dem Teleskopnagel ergeben sich bei der Verankerung seines distalen Teils, da dazu immer ein Gelenk geöffnet werden muß, was eine erhöhte Infektionsgefahr in sich birgt ; darüberhinaus kommt es bei der Verankerung sehr häufig zu Lockerungen des T-Stücks am distalen sowie am proximalen Ende des Nagels.

Aufgabe der Erfindung ist es, ein Langzeit-Implantat — d.h. für Implantatzeiten von mindestens drei Jahren und mehr — zu schaffen, das dem Längen- und dem Dickenwachstum in gewissem Umfang nachgeben und folgen kann, und bei dem darüberhinaus in den Markraum des Knochens nicht eingegriffen werden muß ; weiterhin soll das neue Implantat nicht eine vollständige Entlastung des Knochens bewirken, sondern diesen lediglich stützen und sein Wachstum führen. Diese Aufgabe wird gemäß der Erfindung durch die in Anspruch 1 gennanten Merkmale gelöst.

Von den Stützschienen des neuen Implantats werden dabei mehrere — im allgemeinen zunächst drei —, außen über den Umfang verteilt, an dem Knochen befestigt ; sie sind in dem mit Vorteil als einzelne Briden ausgebildeten, auf dem Knochenmantel verankerten Halterungen so gehalten und geführt, daß sie relativ zu den Halterungen und damit zu dem Knochen verschiebbar bleiben. Zum einen wird dadurch das erwünschte Nachgeben des Implantats dem Längenwachstum gegenüber gewährleistet und zum anderen erreicht man dabei auch, daß Belastungen sehr weitgehend von dem Knochengewebe aufgenommen werden müssen, dieses also von seinen natürlichen Funktionen so wenig wie möglich entlastet wird.

Ihre Querschnittsform bewirkt dabei, daß die Schiene mindestens zunächst nur mit den vorzugsweise abgerundeten Enden der Abstützflächen auf dem Knochen aufliegt. Durch das den Umfang vergrößernde Dickenwachstum wird dann von der diesem folgenden Bride — wegen der durch das Wachstum hervorgerufenen Vergrößerungen des Abstandes ihrer Verankerungspunkte auf dem Knochenmantel — eine radial nach innen wirkende Kraft auf den Scheitel der Falte der Schiene ausgeübt. Dieser Kraft gibt die Schiene durch Aufspreizen der Falte nach. Um die Wirkung der vom Dickenwachstum ausgeübten nach innen gerichteten Radialkraft möglichst gleichmäßig auf den Scheitel der Falte auszurichten, kann man die Halterungen mit « Soll »-Verformungsstellen ausstatten, in denen sie beim Auseinanderwandern ihrer Fixpunkte auf dem Knochenumfang in einer gewünschten Richtung abgebogen werden.

Im Laufe der Zeit wird ein Wachstum des Knochens um die Schiene herum erfolgen, wodurch diese dann zusätzlich in eine Art « Führungen » des sie umgebenden Knochens eingebettet und gegen seitliche Verschiebungen gesichert werden.

Die Formgebung des Schienenquerschnitts bringt darüberhinaus den zusätzlichen Vorteil, daß der Widerstand der Schiene gegen ein Verbiegen aus ihrer Längsrichtung hinaus relativ groß ist.

Da das neue Implantat dem Wachstum in weiten Grenzen zu folgen vermag, ist es möglich, es bereits nach der ersten Spontan-Fraktur und/oder zu Beginn des den Knochen verkrümmenden Wachstums zu implantieren; selbstverständlich kann man jedoch auch durch Osteotomie korrigierte, zuvor bereits stark verkümmte Knochen damit behandeln.

Als weiterer Vorteil des neuen Implantats ist zu erwähnen, daß Reoperationen erst nach mehreren Jahren erforderlich sind und daß es bei diesen darüberhinaus nicht notwenig ist, die bis daher vorhandenen Implantatteile zu entfernen und zu ersetzen, sondern daß man diese Implantate nur durch zusätzliche Schienen ergänzen muß, die entweder in Verlängerung der vorhandenen Schienen oder in Umfangsrichtung zwischen den bisherigen Implantaten angeordnet sein können.

Weiterhin führen die einfache Montage auf dem äußeren Umfang des Knochens sowie die genannten Vorteile dazu, daß die Operationstechnik — besonders gegenüber derjenigen bei Marknagel-Implantationen — stark vereinfacht und erleichtert wird, wodurch die Schwere der Eingriffe geringer und unter Umständen die Hospitalisierungszeit der Kranken verkürzt werden.

Als Werkstoff für Schienen und Halterungen dienen in der Implantattechnik übliche Metalle, während das mit Vorteil relativ elastische und nachgiebige Zwischenstück aus knochenverträglichem Kunststoff, insbesondere aus Polyäthylen der Kategorien HDPE oder UHMW besteht.

Im folgenden wird die Erfindung anhand eines Ausführungsbeispiels im Zusammenhang mit der Zeichnung näher erläutert.

Fig. 1 ist eine Skizze eines mit dem neuartigen Implantat versehenen Oberschenkelknochens,

Fig. 2 zeigt in einer vergrößerten, auseinandergezogenen Darstellung eine Aufsicht auf die Einzelelemente — Schiene, Zwischenstück und Halterung — des neuen Implantats, gesehen in Richtung der Längsachse seiner Stützschiene, während

Fig. 3 in einem Ausschnitt einen Schnitt III-III durch den Knochen nach Fig. 1 im Bereich einer Halterung wiedergibt.

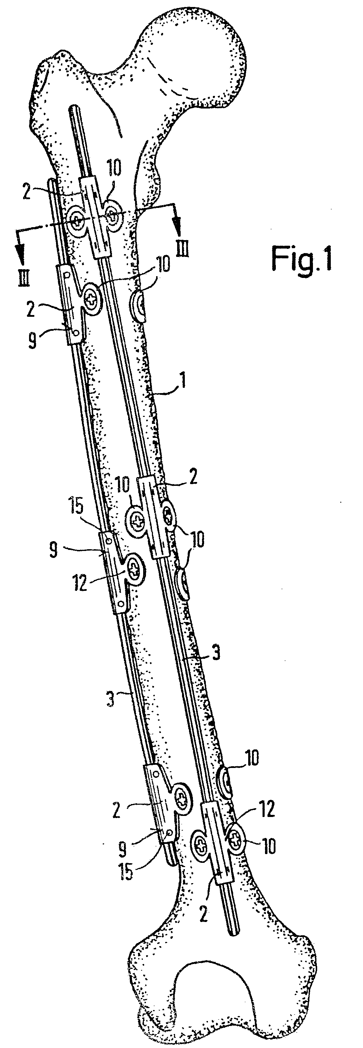

Auf dem Umfang des zu stützenden Knochens 1 (Fig. 1) sind mit Hilfe von Halterungen 2, die im Knochenmantel beispielsweise mit Schrauben befestigt sind, drei Stützschienen 3 angeordnet. Jede Schiene ist im gezeigten Beispiel durch drei Halterungen 2 gehalten, die aus einzelnen, jeweils nur einen Teil des Umfangs umfassenden Briden 9 bestehen, um ein möglichst ungehindertes Dickenwachstum eines Knochens 1 zu gewährleisten. Die Halterungen 2 sind zudem aus Platzgründen in Längsrichtung des Knochens versetzt zueinander angeordnet.

Wird der zu stützende Knochen 1 vor der Anbringung der Implantate mit Hilfe einer Osteotomie korrigiert, so ist die Stützschiene an jedem einzelnen Teilstück durch mindestens eine Halterung 2 gehalten und geführt.

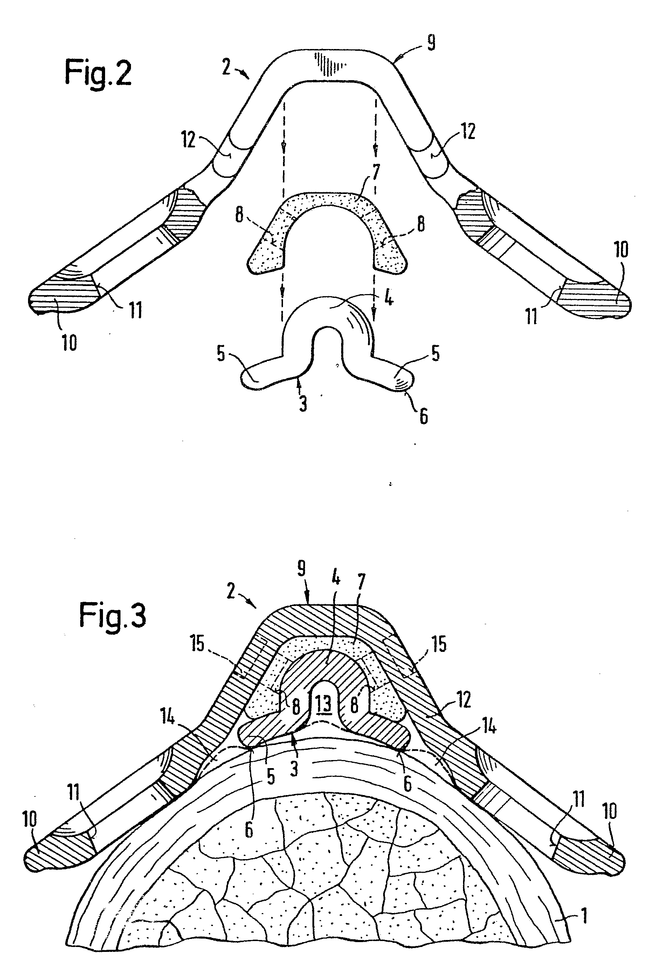

Wie die vergrößerte Darstellung der Fig. 2 erkennen läßt, ist die aus einem Metallblech gefertigte Schiene 3 in ihrem Mittelteil 4 U-förmig gefaltet. Die Schenkel gehen nach außen in Abstützflächen 5 über, die abgewinkelt und unter Umständen zusätzlich gekrümmt sind. In den zur Aufnahme auf dem Knochen 1 bestimmten Bereichen sind die Abstützflächen abgerundet und abgeflacht.

Im Bereich der Halterungen 2 ist die Außenseite der Falte 4 der Schiene 3 umgeben von einem Zwischenstück 7, das, wie erwähnt, aus Kunststoff besteht. Dieses ruht mit seinen seitlichen Flanken auf den Außenseiten der Abstützflächen 5. Seine Flanken sind darüberhinaus mit Bohrungen oder Vertiefungen 8 versehen, in die zu seiner Fixierung in der als Bride 9 ausgebildeten Halterung 2 deren Wand mit einem Werkzeug eingedrückt werden kann (Ziffer 15 in Fig. 3).

Die Bride 9, die mit ihrem Mittelteil das Zwischenstück 7 und die Schiene 3 umfaßt (Fig. 3), endet auf beiden Seiten in Laschen 10, in denen Bohrungen 11 für den Durchtritt der nicht dargestellten Schraubenbolzen vorgesehen sind, mit denen die Bride 9 in dem Knochenmantel verschraubt werden kann. Im Bereich der Nahtstelle zwischen dem Mittelteil und den Laschen 10 ist auf beiden Seiten — beispielsweise durch eine Verringerung der Breite der Lasche — eine « Soll-Biege-Linie » 12 vorgesehen, entlang oder die Bride 9 sich beim Wachstum des Knochens 1 verbiegen soll, um in gewissem Umfang der Abstandsvergrößerung ihrer Fixierungspunkte auf dem Knochumfang nachzugeben.

Wie besonders Fig. 3 zu entnehmen ist, sind die Außeneiten der Schiene 3, die Form des Mittelteils der Bride 9 und Form und Größe des Zwischenstücks 7 so aufeinander abgestimmt, daß das Element 7 nach der Befestigung des Implantats am Knochen 1 praktisch vollständig von den Metallteilen 3 und 9 umgeben und gefangen ist. Durch dieses Einschließen wird ein Kaltfließen des Zwischenstücks 7 als Folge der durch das Dickenwachstum auftretenden radialen Kräfte möglichst verhindert.

Wie weiterhin aus Fig. 3 ersichtlich, sind die Abwinklung und/oder Krümmung der Abstützfläche 5 zum Zeitpunkt der Implantation derart, daß die Schiene 3 zunächst nur mit ihrer abgerundeten Fläche 6 auf dem Knochen 1 aufliegt. Im Laufe des Wachstums wird der Knochen 1 in die im Implantat vorhandenen Hohlräume 13 bzw. 14 einwachsen, was in Fig. 3 gestrichelt dargestellt ist; dadurch entstehen für die Abstützflächen 5 der Schiene 3 bahnartig vertiefte Führungen, die ein seitliches Auswandern und Verschieben der Schiene 3 zumindest erschweren. Weiterhin wird durch die bereits mehrfach erwähnte radiale Kraft auf den Scheitel der Falte 4 die Schiene 3 auf den Knochen 1 gepreßt. Sie reagiert durch elastisch federndes Nachgeben der Abstützflächen 5 auf diese Kraft, wobei sie mit immer größeren Anteilen der von den Stellen 6 auf der Innenseite zur Falte 4 hin verlaufenden Oberflächen ihrer Abstützflächen 5 an dem Knochen 1 zur Anlage kommt.

## Ansprüche

1. Dem Längen- und Dickenwachstum nachgebendes Implantat zur Stützung von Röhrenknochen des menschlichen Skeletts, gekennzeichnet durch eine im wesentlichen in Achsrichtung des zu stützenden Knochens (1) verlaufende Stützschiene (3) aus Metallblech, deren Querschnitt in seinem zentralen Bereich zu einer nach außen hervorstehenden Falte (4) zusammengebogen ist, an die sich zu beiden Seiten flügelartige Abstützflächen (5) zur äußeren Anlage an dem Knochenmantel anschließen, wobei diese Flächen derart abgewinkelt und/oder gekrümmt sind, daß mindestens zum Zeitpunkt der Implantation das Implantat stärker gekrümmt bzw. abgewinkelt ist als der Knochenmantel, und ferner gekennzeichnet durch mit dem Knochen fest verbindbare Halterungen (2), durch die die Schiene (3) mindestens an ihren Enden mit Hilfe eines Zwischenstücks (7) aus Kunststoff verschiebbar gehalten und geführt ist.

2. Implantat nach Anspruch 1, dadurch gekennzeichnet, daß die Abstützflächen (5) der Schiene (3) im Bereich der Anlage an dem Knochen (1) abgerundet sind.

3. Implantat nach Anspruch 1, dadurch gekennzeichnet, daß die Halterungen (2) mit Mitteln (12) für Verformungen an vorgegebenen Stellen längs gewünschter Richtungen versehen sind.

## Claims

1. An implant which yields to growth in length and thickness and which is intended for supporting tubular bones of the human skeleton, characterised by a sheet-metal supporting rail (3) extending substantially in the axial direction of the bone (1) requiring support, the central region of the cross-section of said rail being bent to form an outwardly projecting fold (4) adjoining which there are wing-like bearing surfaces (5) on either side for external contact with the outer surface of the bone, the said surfaces being so bent and/or curved that the implant has a greater curvature or bend than the outer surface of the bone, at least when the implant is implanted, and further characterised by retainers (2) which are adapted to be rigidly connected to the bone and by means of which the rail (3) is slidably held and guided, at least at its ends, by means of a plastics intermediate member (7).

2. An implant according to claim 1, characterised in that the bearing surfaces (5) of the rail (3) are rounded in the region where they contact the bone (1).

3. An implant according to claim 1, characterised in that the retainers (2) are provided with means (12) for deformation in required directions at predetermined places.

## Revendications

1. Elément d'implantation capable de céder à la croissance en longueur et en épaisseur et destiné à soutenir un os long du squelette humain, caractérisé en ce qu'il comprend une barre d'appui (3) formée d'une feuille de métal, orientée sensiblement dans la direction de l'axe de l'os devant être soutenu (1) et dont la section transversale de sa partie centrale est recourbée de manière à former un repli (4) en saillie vers l'extérieur et prolongé des deux côtés par des surfaces d'appui (5) formant des ailes et destinées à reposer extérieurement sur l'enveloppe de l'os, ces surfaces étant coudées et/ou incurvées de manière que l'élément d'implantation soit plus fortement courbé ou coudé que l'enveloppe de l'os, au moins au moment de l'implantation de cet élément, des supports (2) destinés à être fixés sur l'os retenant et guidant la barre (3) de manière qu'elle puisse coulisser, au moins à ses extrémités, par l'intermédiaire d'une pièce intercalaire (7) de matière plastique.

2. Elément d'implantation selon la revendication 1, caractérisé en ce que les surfaces d'appui (5) de la barre (3) sont arrondies à l'emplacement auquel elles sont destinées à reposer sur l'os (1).

3. Elément d'implantation selon la revendication 1, caractérisé en ce que les supports (2) comportent des éléments (12) autorisant des déformations en des emplacements prédéterminés le long de directions voulues.

0 007 393

1/2

Fig.1

Fig.2

Fig.3